# EUROPEAN PATENT APPLICATION

(11) **EP 2 186 810 A1**
(43) Date of publication of application: **19.05.2010**
(21) Application number: 08168104.1
(22) Date of filing: 31.10.2008
(51) Int. Cl.: C07D 413/14, A61K 31/444, A61P 35/00

(54) **Poly-heteroaryl derivatives for the treatment of cancer**

(71) Applicant: Institut Curie, 75248 Paris Cedex 05 (FR); Centre National de la Recherche Scientifique, 75794 Paris Cedex 16 (FR); INSTITUT NATIONAL DE LA SANTE ET DE LA RECHERCHE MEDICALE (INSERM), 75654 Paris Cédex 13 (FR); MUSEUM NATIONAL D'HISTOIRE NATURELLE, 75231 Paris Cedex 05 (FR)
(72) Inventor: Nguyen, Chi-Hung, F-92160 Antony (FR); Rouchon Dagois, Myriam, F-91400 Orsay (FR); Guedin-Baurepaire, Aurore, F-91240 Saint-Michel-sur-Orge (FR); Monchaud, David, F-21000 Dijon (FR); Teulade-Fichou, Marie-Paule, F-75014 Paris (FR); Riou, Jean-François, F-75014 Paris (FR); Mergny, Jean-Louis, F-33400 Talence (FR); Grierson, David, CA-Vancouver BC, V6T 2L1 (CA)
(74) Representative: Peaucelle, Chantal

(57) **Abstract**

The polyheteroaryl derivatives of the invention are penta-, hexa-, hepta-, octa-, nona- and deca-heteroaryl (abbreviated hereafter as penta- to deca-heteroaryl) structurally are characterized by a combination of heterocycle 1 (Het-1)ₐ and/or heterocycle 2 (Het-2)_{b} and/or heterocycle 3 (Het-3)_{c} and/or heterocycle 4 (Het-4)_{d} of formulae I, II, III and IV respectively, 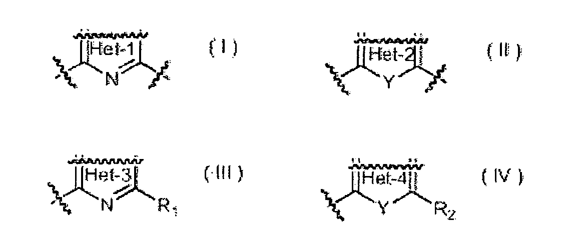 the N-oxides, the pharmaceutically acceptable addition salts.
The compounds claimed are suitable for anti-cancer agents.

## Description

The present invention relates to poly-heteroaryl derivatives that specifically bind and stabilise quadruplex DNA. It also relates to the synthesis thereof and their biological applications.

G-quadruplex DNA is currently considered as a structural element able to regulate the function of G-rich sequences. In particular, many lines of evidence suggest that formation of this peculiar DNA structure at telomeres or in specific gene promoters in a variety of human oncogenes, including c-Myc, Bcl-2, VEGF, Hif-1a, Ret, c-Kit, PDGF-A, KRAS and c-Myb (1) may inhibit cancer cell proliferation.

Telomeric DNA of human cells comprises tandem repeats of sequence 5'-TTAGGG-3', which terminates on their 3' side in a single-stranded overhang that was demonstrated to fold into G-quadruplex *in vitro.* The formation of such quadruplex-structure *in vivo* is hypothesized to lead to the displacement of protective proteins normally associated with telomeres (shelterin complex), therefore disrupting telomere structure leading to genomic instability.

For oncogenes promoters, stabilisation of quadruplex is expected to disrupt the local environment of proteins factors that regulates gene expression and has been well documented for c-myc (1). Hence chemical intervention able to induce or stabilize quadruplex-DNA formation has been thoroughly investigated *via* small molecules quadruplex binders, with the goal to control DNA-related functions (telomere elongation, oncogene transcription) in particular in cancer cells.

Compounds that interact with G-quadruplex-DNA are currently numerous in the literature, as illustrated by the number of recent reviews dedicated to them (2- 9) However only few compounds present a high degree of selectivity for quadruplex- *vs* duplex-DNA; additionally, only a few of these compounds have been thoroughly investigated as anti-cancer agents. The leading compound of this family is telomestatin, a naturally-occurring macrocycle (isolated from *Streptomyces anulatus* 3533-SV4) comprised of five oxazole rings, two methyl oxazole rings and a thiazoline ring. Telomestatin represents a paradigm in terms of quadruplex-selective interactions, and shows exceptional telomerase-inhibiting properties *in vitro* (evaluated through either TRAP or Direct assay, with IC₅₀-TRAP = 0.6nM and IC₅₀Direct Assay = 58nM (10). Additionally, telomestatin is also investigated for its cellular effects and presents antiproliferative and apoptotic properties in various tumor cell lines (11 and 12). Telomestatin alters telomere integrity (13) and triggers a DNA-damage response at telomeres (14). Telomestatin induced POT1 uncapping *in vitro* (IC_{50-POT1} = 500 nM) and removes GFP-POT1 from telomeres in tumor cells (15). Given the harsh synthetic access of telomestatin (16), analogues have been reported (17, 18 and 19) that present interesting quadruplex-interacting properties.

However, none of these compounds show the exceptional properties of telomestatin. Additionally, none of these compounds, including telomestatin itself, have been investigated for their fluorescence properties. Given the chemical instability, the poor water-solubility and in particular, the arduous synthesis of telomestatin, its large-scale therapeutic use is questionable.

In this context, the inventors have found that new non-macrocyclic polyheteroaryl derivatives exhibit an exquisite selectivity for quadruplex- over duplex-DNA and that the association of these derivatives with their DNA targets i) leads to deep modifications of their spectroscopic properties, ii) uncap human POT1 (protection of telomere 1) protein from the telomeric G-overhang, iii) inhibit cell proliferation of human tumour cell lines. The stability and solubility in physiological media of this new family of compounds are compatible with their use in therapeutic treatments. Moreover, they have found an efficient and original process for obtaining these compounds.

An object of the present invention is then to provide, as new products, such non-macrocyclic polyheteroaryl derivatives.

It also relates to a method for their synthesis.

According to another object, the invention taking advantage of the quadruplex-interacting properties of said compounds, further relates to the use of said derivatives as quadruplex-specific probes.

In still another object, the invention provides pharmaceutical compositions containing said derivatives as active principles and also relates to the use of said derivatives in the manufacture of drugs useful for the treatment of patients suffering from genetic disorders, for example cancer, or infectious diseases such as malaria.

The invention also relates to a method for treating cancer or infections diseases comprising administering an efficient amount of said derivatives to a patient in need thereof

The polyheteroaryl derivatives of the invention are penta-, hexa-, hepta-, octa-, nona- and deca-heteroaryl (abbreviated hereafter as penta- to deca-heteroaryl)structurally characterized by a combination of heterocycle 1 (Het-1)ₐ and/or heterocycle 2 (Het-2)_{b} and/ or heterocycle 3 (Het-3)_{c} and/or heterocycle 4 (Het-4)_{d} of formulae I, II, III and IV respectively, 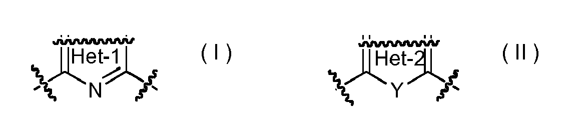 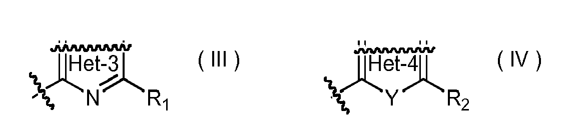 the N-oxides, the pharmaceutically acceptable addition salts, wherein
- a, b, c and d are integers from 0 to 3, the sum a + b + c + d being ≤ 10
- Y is O or S;
- Het-1 is a class of nitrogen heterocyclic-diyl ring selected in the group comprising 2,6-pyridin-diyl or 2,4-pyrimidin-diyl or 3,5-pyrazin-diyl or 2,4-(1,3,5-triazin)diyl or 3,5-(1,2,4-triazin)diyl or 2,4-oxazolin-diyl or 2,4-thiazolin-diyl , optionnally being substituted with one, two or three substituents, each independently selected from C₁₋₆alkyl ; C₁₋₄alkyloxy ; C₁₋₆alkyloxycarbonyl ; carboxy ; formyl ; halo ; cyano ; azido ; hydroxy ; nitro ; amino ; mono-or di(C₁₋₄alkyl)amino ; C₁₋₄alkylmethylamino ; morpholin-4-ylC₁₋₄alkyloxy; piperazin-1-ylC₁₋₄alkyloxy ; 4-C₁₋₄alkylpiperazin-1-ylC₁₋₄alkyloxy; monocyclic or bicyclic ring selected from phenyl, benzyl, naphthyl ;
- Het-2 is a class of five-membered heterocyclic-diyl ring selected in the group comprising 2,5-oxazolin-diyl or 2,5-thiazolin-diyl, or 2,5-thiophen-diyl or 2,5-furan-diyl, optionally being substituted with one, or two substituents each independently selected from C₁₋₆alkyl ; C₁₋₄alkyloxy ; C₁₋₆alkyloxycarbonyl ; carboxy ; formyl ; halo ; cyano ; azido ; hydroxy ; nitro ; amino ; mono-or di(C₁₋₄alkyl)amino ; C₁₋₄alkylmethylamino ; morpholin-4-ylC₁₋₄alkyloxy ; piperazin-1-ylC₁₋₄alkyloxy; 4-C₁₋₄alkylpiperazin-1-ylC₁₋₄alkyloxy; monocyclic or bicyclic ring selected from phenyl, benzyl, naphthyl ;
- Het-3 and/ or Het-4 constitutes the endings of the said penta- to deca-heteroaryl derivatives, wherein;
   - Het-3 is a class of nitrogen heterocyclic-yl ring selected from 2-pyridyl or 2-pyrimidyl or 4-pyrimidyl or 2-pyrazyl or 2-(1,3,5)triazyl or 3-(1,2,4)triazyl or 5-(1,2,4)triazyl or 2-oxazolyl or 4-oxazolyl or 2-thiazolyl or 4-thiazolyl optionally being substituted with one, two or three substituents,each independently selected from C₁₋₆alkyl ; C₁₋₄alkyloxy ; C₁₋₆alkyloxycarbonyl ; carboxy ; formyl ; halo ; cyano ; azido; hydroxy ; nitro ; amino ; mono-or di(C₁₋₄alkyl)amino ; C₁₋₄alkylmethylamino ; morpholin-4-ylC₁₋₄alkyloxy ; piperazin-1-ylC₁₋₄alkyloxy ; 4-C₁₋₄alkylpiperazin-1-yl C₁₋₄alkyloxy ; monocyclic or bicyclic ring selected from phenyl, benzyl, naphthyl ;
   - Het-4 is a class of five-membered heterocyclic-yl ring selected from 2-oxazolyl or 5-oxazolyl or 2-thiazolyl or 5-thiazolyl or 2-thienyl or 2-furanyl optionally being substituted with one or two substituents each independently selected from
   - C₁₋₆alkyl ; C₁₋₄alkyloxy ; C₁₋₆alkyloxycarbonyl ; carboxy ; formyl ; halo ; cyano ; azido ; hydroxy ; nitro ; amino ; mono-or di(C₁₋₄alkyl)amino ;
   - C₁₋₄alkylmethylamino ; morpholin-4-ylC₁₋₄alkyloxy; piperazin-1-ylC₁₋₄alkyloxy; 4-C₁₋₄alkylpiperazin-1-ylC₁₋₄alkyloxy; monocyclic or bicyclic ring selected from phenyl, benzyl, naphthyl ;
- R₁ and R₂, are each independently selected from hydrogen; C₁₋₆alkyl ; C₁₋₄alkyloxy ; C₁₋₆alkyloxycarbonyl ; carboxy ; formyl ; halo ; cyano ; azido ; hydroxy ; nitro ; amino ; mono-or di(C₁₋₄alkyl)amino ; C₁₋₄alkylmethylamino ; monocyclic or bicyclic ring selected from phenyl, benzyl, naphthyl optionally being substituted with one, two or three substituents each independently selected from halo, hydroxy, C₁₋₄alkyl, C₁₋₄alkyloxy, morpholin-4-yl C₁₋₄alkyloxy, piperazin-1-ylC₁₋₄alkyloxy, 4-C₁₋₄alkylpiperazin-1-yl C₁₋₄alkyloxy or phenyl.

In an advantageous group, in view of the G-quadruplex interacting properties of the derivatives, Het-1 is linked to two Het-2.

In preferred derivatives of said group, Het-1 is a pyridyl and Het-4 is an oxazolyl, substituted by a pyridyl or a pyridyl-oxazolyl.

In a second advantageous group, Het-2 is linked to two Het-1.

In preferred derivatives, Het-2 is an oxazolyl and Het-1 is a pyridyl oxazolyl.

As shown in the Examples hereinafter, the derivatives of the invention specifically bind and stabilise quadruplex DNA and therefore are of great interest as quadruplex-specific probes.

They are also of great value for the treatment of various diseases.

The invention thus relates to the use of the above defined derivatives as drugs.

It also relates to pharmaceutical compositions comprising an effective amount of at least one derivative such as above defined in combination with a pharmaceutically acceptable carrier.

Said pharmaceutical compositions are optionally in association with other active principles.

The above compositions are advantageously under forms suitable for an administration by the oral or parenteral route.

Forms appropriate for an oral administration comprise tablets, pills.

For a parenteral use, the compositions are under the form of solutions.

Said compositions are particularly useful to treat cancer and infectious diseases such as malaria.

In general, an effective daily amount of active principle in the compositions will be from 0.01 mg/kg to 50 mg/kg body weight, more preferably from 0.1 mg/kg to 10 mg/kg body weight.

The use of the above derivatives for making a drug for treating cancer or infectious disease enters into the scope of the invention.

The invention also covers a method for making the above defined derivatives.

Said method comprises
- reacting X1-(di- or tri- or tetra- heteroaryl-1,4)-X2
- with a (mono- or di- or tri- or tetra- heteroaryl-1,4)-X3
wherein
- Heteroaryl-1,4 is a combination of Het-1 and/or Het-2 and/or Het-3 and/or Het-4 which are as defined above,
- X1 and X2, identical or different, represent an hydrogen or halogen or triflate group,
- X3 represents an halogen or zinc halogenide or trialkyltin or boronate group
under conditions giving the desired polyheteroaryl derivatives.

Other characteristics and advantages of the invention are given in the following Examples, which refer to Fig.1 to Fig. 15, wherein
- Fig.1A- Fig.1D represents absorption spectra of a derivative according to the invention in various conditions;
- Fig.2, the various titrations summary;
- Fig.3A-Fig3B results of FRET-melting assay with the oligonucleotide mimicking the human telomeric sequence (F21T) with or without the derivative of invention in Na+ or K+ conditions.
- Fig.4 and Fig.5A-Fig5B, results of FRET-melting of G-quadruplex with or without a derivative of the invention and in the presence of duplex- or quadruplex-DNA competitors
- Fig.6, the summary of the various FRET-melting experiments;
- Fig.7A and Fig.7B, results of fluorescence titrations with a derivative of the invention with addition of quadruplex-DNA in Na+ and K+ conditions.
- Fig.8, fluorescence titration results when using a quadruplex or using a short-duplex DNA with addition of a derivative of the invention;
- Fig.9, fluorescence titrations results when using various quadruplex-DNA structures with addition of a derivative of the invention;
- Fig.10, a graphical representation of the summary of the various fluorescence titrations;
- Fig.11A and Fig.11B and Fig.12, results of circular dichroism of quadruplex-DNA with addition of a derivative of the invention;
- Fig. 13, results of circular dichroism relating to quadruplex-structure induction with a derivative of invention
- Fig. 14, results relating to the inhibition of POT1 binding to telomeric sequence in vitro by a derivative according to the invention;
- Fig.15A-Fig15B results relating to the inhibition of cancer cell proliferation (two cell lines) by a derivative according to the invention.

### I - Synthesis of poly-heteroaryl derivatives

The following examples are intended to illustrate the present invention. The compounds can also obtained by other processes known by the man skilled in the art.
The starting materials used 2,6-pyridine dicarboxaldehyde, 6-bromopyridine-2-carbaldehyde, *p*-toluenesulfonylmethyl isocyanide (TosMIC) and 2-pyridylzinc-bromide, are commercially available.

### 1. : Example 1: 2,6-Bis[2-(pyridin-2-yl)oxazol-5-yl]pyridine (Compound 1)

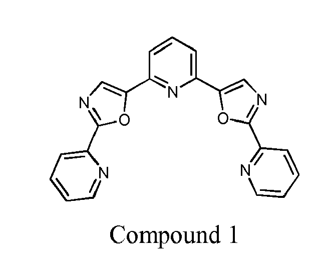

### 1.1.: 2,6-Bis(oxazol-5-yl)pyridine (intermediate 1)

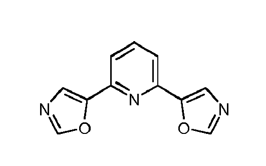

2,6-Pyridine dicarboxaldehyde (4.0 g ; 29.2 mmol), TosMIC (11.4 g ; 58.3 mmol) and potassium carbonate (16. 3 g ; 117.8 mmol) in 100 mL of methanol were heated at reflux during 3 hours. The solvent was evaporated in vacuo and the residue was poured into brine solution and extracted with dichloromethane (4x150 mL). The combined organic layers were dried over magnesium sulfate and concentrated in vacuo. The residue was purified by flash chromatography on silica gel column with dichloromethane-ethanol (95-5) as the eluent to give the titled compound as yellow solid (5.6 g, 90%), m.p.= 190-191°C; ¹H NMR (300 MHz, CDCl3) 7.56 (d ; 2H ; J= 8.0) ; 7.74 (s ; 2H) ; 7.80 (t; 1H; J= 8.0 Hz) ; 7.96 (s ; 2H) ; ¹³C NMR (75 MHz, CDCl3) 118.7, 125.7, 138.0, 147.4, 150.8, 151.0; SM *m*/*z* 214.1 (M+1); Anal. calcd for C₁₁H₇N₃O₂: C, 91.97 ; H, 3.31 ; N, 19.71. found: C, 61.51, H, 3.43, 19.48.

### 1.2.: 2,6-Bis-(2-iodooxazol-5-yl)pyridine (intermediate 2)

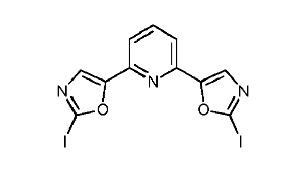

Under an argon atmosphere, the intermediate 1 (0.1 g ; 0.5 mmol) and TMEDA (0.2 mL ; 1.0 mmol) are dissolved in 5 mL of anhydrous THF and cooled at -78°C. A solution of LiHMDS 1M in THF (1.0 mL ; 1.0 mmol) was added dropwise and stirred during 30 minutes at -78°C and one hour at -40°C. The mixture was cooled again at -78°C and 1,2-diiodoethane (0.5 g ; 1.9 mmol) was added. After stirring over night at room temperature the mixture was poured into a sodium thiosulfate solution and extracted with ethyl acetate (3x10 mL). The combined organic layers were dried over magnesium sulfate and concentrated in vacuo. The residue was purified by flash chromatography (SiO₂, dichloromethane-ethanol, 95-5) to give the intermediate 2 as beige solid (117 mg, 50%), ¹H NMR (300 MHz, CDCl3) 7.58 (d ; 2H ; J= 8 Hz); 7.69 (s ; 2H); 7.85 (t; 1H ; J= 8 Hz); ¹³C NMR (75 MHz, CDCl3) 119.5, 129.7, 138.7, 147.0, 157.2, 151.5; SM *m*/*z* 465.8 (M+1), 487.8 (M+23).

### 1.3.: 2,6-bis[2-(pyridin-2-yl)oxazol-5-yl]pyridine (Compound 1)

Method 1 : A mixture of intermediate 2 (65 mg ; 0.14 mmol), 2-pyridylzinc-bromide (1.2 mL ; 0.60 mmol) and Pd(PPh₃)₄ (12 mg ; 0.01 mmol) were heated at reflux in 2 mL of anhydrous THF during 4 hours. Water was added and the mixture was extracted with ethyl acetate (3x10 mL). The combined organic layers were dried over magnesium sulfate, filtered and concentrated *in vacuo.* The residue was purified by flash chromatography (SiO₂, dichloromethane-ethanol, 95-5) to give the expected compound 1 as beige solid (13 mg, 25%), m.p.=242-245°C; ¹H NMR (300 MHz, CDCl3) 7.40 (m ; 2H) ; 7.83-7.88 (m ; 5H) ; 7.97 (s ; 2H) ; 8.23 (d ; 2H ; J= 7.9 Hz) ; 8.77 (d ; 2H ; J= 4.5 Hz) ; ¹³C NMR (75 MHz, CDCl3) 119.7, 123.3, 125.6, 128.6, 137.8, 138.5, 146.6, 147.9, 150.9,152.3, 161.5; SM *m*/*z* 368.0 (M+1), 390.0 (M+23).

Method 2 : A mixture of intermediate 1 (0.1 g ; 0.5 mmol), 2-bromopyridine (91 µL ; 0.9 mmol), palladium diacetate (11 mg ; 0.05 mmol ; 10 mol%), PCy₃.HBF₄ (35 mg ; 0.1 mmol ; 20 mol%), copper (I) iodide (0.18 g ; 0.9 mmol), cesium carbonate (0.61 mg ; 1.88 mmol) and 1.3 mL of anhydrous toluene were submitted under microwave irradiation conditions (130°C, 150W) during 4 hours. The residue was purified by flash chromatography (SiO₂, dichloromethane-ethanol, 95-5) to give the compound 1 (8 mg, 33%) which is identically with than that described in method 1 above.

### 2. Example 2 : 2,5-Bis-[6-(oxazol-5-yl)]pyridin-2yloxazole (Compound 2)

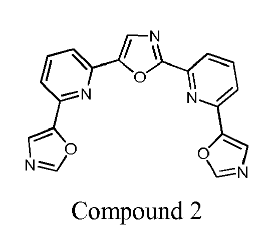

### 2.1: 5-(2-Bromopyridin-2-yl)oxazole (intermediate 3)

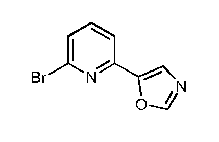

6-Bromopyridine-2-carbaldehyde (4.0 g ; 21.5 mmol), TosMIC (4.2 g ; 21.5 mmol) and potassium carbonate (6.0 g ; 43.4 mmol) were heated at reflux during 3 hours in 75 mL of methanol. The solvent was evaporated in vacuo and the residue was poured into brine solution and extracted with dichloromethane (4x100 mL). The combined organic layers were dried over magnesium sulfate and concentrated in vacuo. The residue was purified by flash chromatography (SiO₂, dichloromethane-ethanol, 95-5) to give the intermediate 3 as yellow solid (2.9 g, 60%); m.p.= 90-91°C; ¹H NMR (300 MHz, CDCl₃) 7.38 (dd ; 1H ; J= 3.5 & 5.3 Hz) ; 7.57 (m ; 2H) ; 7.73 (s ; 1H) ; 7.97 (s ; 1H) ; ¹³C NMR (75 MHz, CDCl3) 118.1, 126.4, 127.6, 139.3, 142.4, 148.0, 151.6 ; SM *m*/*z* 225.0 and 227.1 (M+1).

### 2.2: 2,5-Bis-[6-(oxazol-5-yl)]pyridin-2yloxazole (Compound 2)

A mixture of intermediate 1 (0.10 g ; 0.5 mmol), intermediate 3 (0.10 g ; 0.5 mmol), palladium diacetate (11 mg ; 0.05 mmol ; 10 mol%), PCy₃.HBF₄ (35 mg ; 0.09 mmol ; 20 mol%), copper (I) iodide (0.1 g ; 0.5 mmol), cesium carbonate (0.3 mg ; 0.9 mmol) and 1.3 mL of anhydrous toluene were submitted under microwave irradiation conditions (130°C, 150W) during 6 hours. The residue was purified by flash chromatography (SiO₂, dichloromethane-ethanol, 95-5) to give the expected compound 2 as beige solid (7 mg, 36%); ¹H NMR (300 MHz, CDCl₃) 7.70 (d ; 2H ; J= 7.5 Hz) ; 7.80 (d ; 1H; J= 7.5Hz) ; 7.85 (d ; 2H ; J= 7.5 H) ; 7.90 (s ; 1H); 7.93 (d; 2H ; J= 5 H) ; 8.00 (s ; 1H); 8.04 (d ; 1H ; J= 5 Hz); 8.20 (d ; 1H ; J= 7.5 Hz); SM *m*/*z*358.1 (M+1).

### 3. Example 3 : 2,6-bis{2-[6-(oxazol-5-yl)pyridin-2-yl]oxazol-5-yl}pyridine (Compound 3)

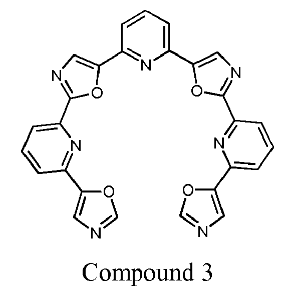

A mixture of intermediate 1 (0.10 g ; 0.5 mmol), intermediate 3 (0.21 g ; 0.9 mmol), palladium diacetate (11 mg ; 0.05 mmoles; 10 mol%), PCy₃.HBF₄ (35 mg ; 0.09 mmol ; 20 mol%), copper (I) iodide (0.18 g ; 0.9 mmol), cesium carbonate (0.61 mg ; 1.9 mmol) and 1.3 mL of anhydrous toluene were submitted under microwave irradiation conditions (130°C, 150W) during 6 hours. The residue was purified by flash chromatography (SiO₂, dichloromethane-ethanol, 95-5) to give the expected compound 3 as beige solid (6 mg, 22%) m.p. >250°C ; ¹H NMR (300 MHz, CDCl3) 7.65 (m; 1H); 7.75 (m; 2H); 7.90-8.05 (m; 9H); 8.10 (s; 1H); 8.20 (m; 2H); SM *m*/*z* 524.2 (M+23).

### II-Biophysical methods and data:

### Study II- 1: UV-vis spectroscopy for investigating solubility properties

### a) Uv-vis titrations:

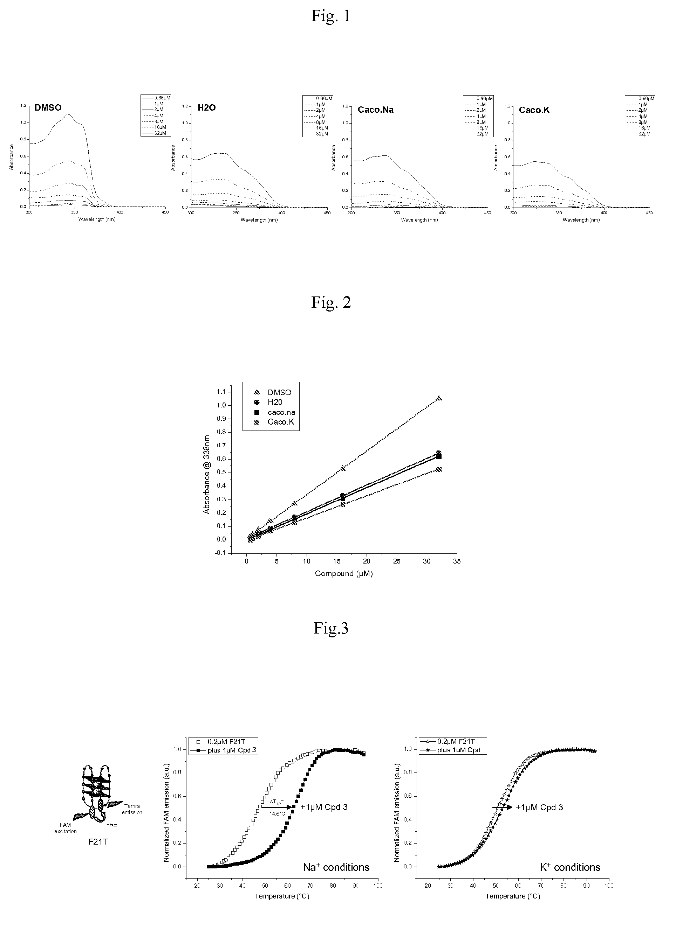

The study of UV-vis properties of pyridine-based polyheteroaromatic compounds detailed in this study enable the determination of their solubility in various conditions (DMSO, H₂O and cacodylate buffers (10mM sodium cacodylate + 100mM NaCl (for Caco.Na) or KCl (for Caco.K)): the simplest method for this is to measure the absorption spectra of **compound 3** at various concentrations (from 0 to 32µM); solubility of **compound 3** is thus evaluated through the reporting of its absorbance at a given wavelength (herein 338nm (A₃₃₈) as a function of the concentration of **compound 3** and applying the Beer-Lambert law. The results are given in Fig.1.

### b) Summary

The various titrations are summarized in Fig.2

These data indicated that **compound 3:**
- is soluble in all the solvent systems used herein; (DMSO, H₂O and aqueous cacodylate buffers (both Na⁺- or K⁺-rich);
- does not self-aggregate within the 0 to 32µM concentration range, in all the solvent systems used herein.

### Study II-2: Stabilization of quadruplex-DNA and quadruplex- over duplex-DNA selectivity

Stabilization, and so interaction, of compounds with quadruplex-structure is monitored *via* FRET-melting assay, in a version that also enables the determination of the quadruplex- over duplex-DNA selectivity as well as the intra-quadruplex selectivity (17).

FRET assay is performed with oligonucleotides that mimic the human or plasmodium telomeric sequences, and equipped with FRET partners at each extremities: F21T (*FAM-*G₃[T₂AG₃]₃-*Tamra*,) FPf1T (FAM-G₃[T₃AG₃]₃-*Tamra*) and FPf8T (*FAM*-G₃[T₂CAG₃]₃-*Tamra*) with *FAM:* 6-carboxyfluorescein and *Tamra*: 6-carboxy-tetramethylrhodamine). Measurements were made with excitation at 492nm and detection at 516nm.

### a) Quadruplex-stabilization:

Fluorescence melting were carried out. The results are given on Fig.3A- Fig.3C. The experiments were carried out with 0.2µM of F21T, FPf1T or FPf8T in a buffer containing 10mM lithium cacodylate pH 7.2 and 100mM NaCl or KCl (see example of F21T below, NaCl (left) and KCl (right)); the melting of the quadruplex-DNA was monitored alone and in the presence of 1µM (5 equiv.) of **compound 3.**

The stabilisation effect induced by compound 3 with the three oligonucleotides is quantified by the increase in melting temperature (ΔT_{1/2}). The values are indicated in the table below.

| ΔT_{1/2} (°C) | F21T | FPf1T | FPf8T |
|---|---|---|---|
| Na⁺ | 14,6 | 20,4 | 16,3 |
| K⁺ | 1,3 | 2,1 | 1,5 |

### b) Quadruplex- vs duplex-DNA selectivity:

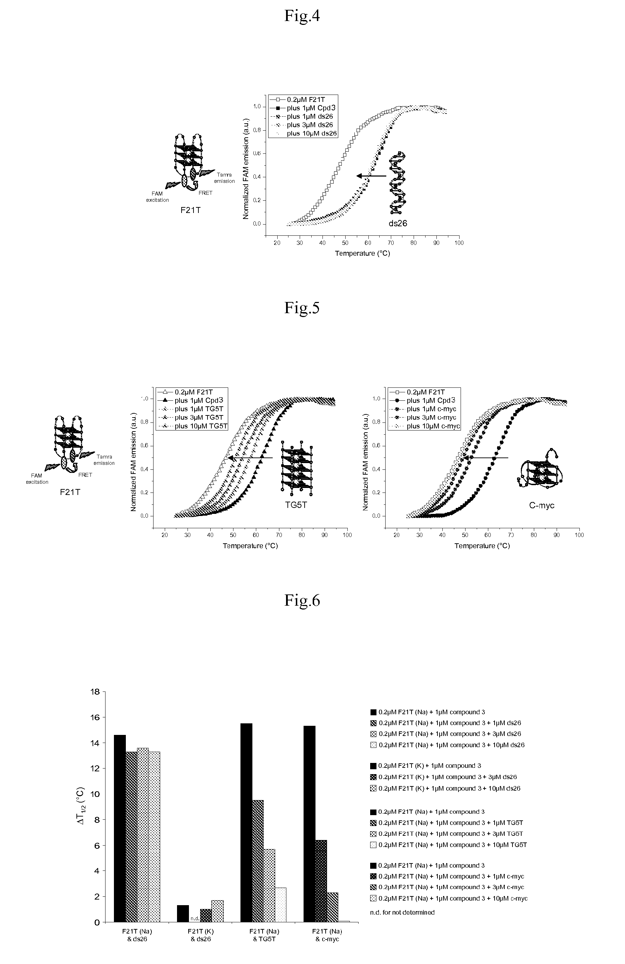

Fluorescence melting are carried out with 0.2µM of F21T in a buffer containing 10mM lithium cacodylate pH 7.2 and 100mM NaCl; the melting of the G-quadruplex was monitored alone and in the presence of 1µM of **compound 3** without or with excess (1, 3 and 10µM) of duplex-DNA competitor ds26 (a 26 base-pair duplex-DNA comprised of the self complementary sequence [5'-CAATCGGATCGAATTCGATCCGATTG-3']). The results are given on Fig. 4

### c) Intra-quadruplex selectivity:

Fluorescence melting experiments are carried out with 0.2µM of F21T in a buffer containing 10mM lithium cacodylate pH 7.2 and 100mM NaCl; the melting of the G-quadruplex was monitored alone and in the presence of 1µM of **compound 3** without or with excess (1, 3 and 10µM) of quadruplex-DNA competitors, either TG5T ([(5'-TG₅T-3')₄], a tetramolecular quadruplex-DNA used in previous study (21) or c-myc ([5'-GAGGGTGGGGAGGGTGGGGAAG-3'], a sequence present in the promoter region of the oncogene c-myc, highly suspected to fold into an intramolecular quadruplex-structure (22-25). The results are given on Fig. 5A -Fig.5B.

### d) Summary

The various FRET-melting experiments are summarized in the graphical bar representation of Fig.6.

These data indicated that **compound 3:**
- interacts efficiently with telomeric quadruplexes F21T (human), FPf1T et FPf8T (*Plasmodium falciparum*) in sodium buffer while it does not stabilize these oligonucleotides in potassium conditions (see Table), meaning that in the three cases examined, **compound 3** presents cation-based intra-quadruplex selectivity;
- discerns very efficiently quadruplex- from duplex-DNA, since F21T stabilization (in sodium buffer) is only marginally affected by the presence of 1, 3 or 10µM of ds26 (the stabilization is >91% maintained in all cases).
- interacts most probably *via* stacking interactions with external G-quartet of quadruplex-structures since the F21T stabilization is highly sensitive to the competition of non-looped tetramolecular quadruplex-DNA (TG5T, F21T stabilization being maintained at 61, 37 and 17% in presence of 1, 3 and 10µM of TG5T respectively) or only-double chain reversal-looped quadruplex-DNA (c-myc, F21T stabilization being maintained at 42, 15 and 1% in presence of 1, 3 and 10µM of c-myc respectively).
- Interaction of the compound both with tetrads and loops can also be hypothesized given the dramatic difference observed between Na⁺ and K⁺ conditions.

### Studs II-3: Interaction with DNA monitored by fluorescence studies

Pyridine-based polyheteroaromatic compounds detailed in this study are characterized by a strong fluorescence. Remarkably, the quantum yield is not affected by the nature of the solvent they are used in, from pure organic (e.g. DMSO) to physiological conditions (e.g. buffer: 10mM sodium cacodylate + 100mM KCl, pH 7.2, see Table). The modification of the fluorescence properties of studied compounds upon interaction with DNA enables to compare their apparent binding affinity for several quadruplexes of biological relevance (27).

| | DCM | Water |
|---|---|---|
| Quantum yield | 0.5 | 0.5 |
| s.d. | 2.17% | 2,89% |

*NB:* the quantum yields (and standard deviations (s.d.)) of **compound 3** were measured in CH₂Cl₂ (DCM) and water, with anthracene in ethanol as reference.

### a) Quadruplex-interaction:

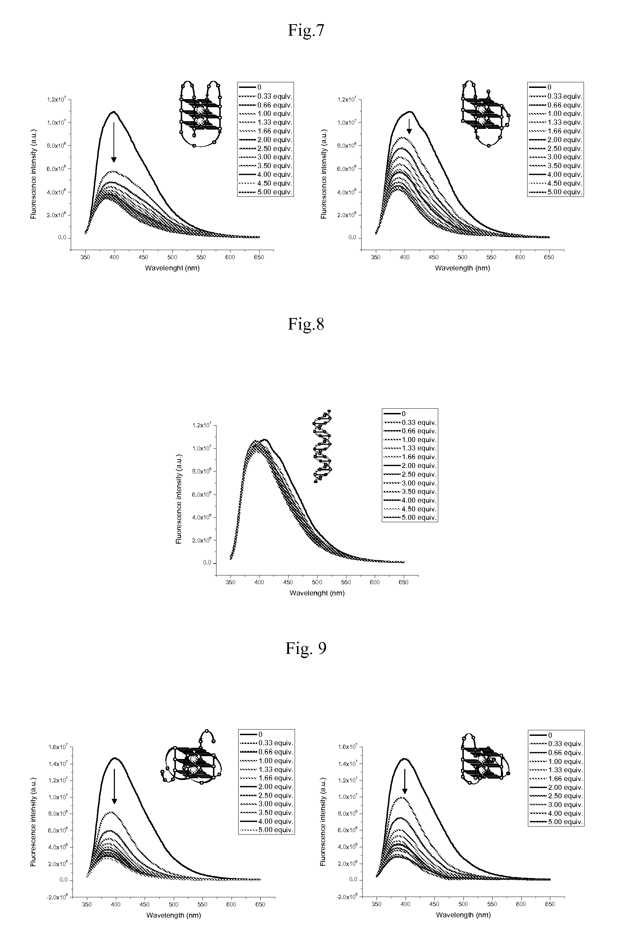

The quadruplex-DNA used herein is 22AG: it results from the folding of a 22nt oligonucleotide that mimics the human telomeric sequence: 22AG is [5'-AG₃(T₂AG₃)₃-3']. Quadruplex-structure from 22AG is prepared by heating the corresponding oligonucleotide at 90°C for 5min in a 10mM sodium cacodylate buffer pH 7.2, 100 mM NaCl (for 22AG Na) or KCl (for 22AG K) and cooling in ice to favor the intramolecular folding by kinetic trapping. Concentrations are determined by UV-Vis measurements (after thermal denaturation, 5min at 85°C) at 260nm before use. (see Fig 7A and Fig.7B).

Increasing amounts of 22AG Na (left) or 22AG K (right) are added onto a solution of0.25µM of **compound 3** in cacodylate buffer (10mM sodium cacodylate + 100mM NaCl (right) or KCl (left)), which result in a progressive quench of the fluorescence of **compound 3** (λₑₓ = 340nm).

### b) Quadruplex- vs duplex-DNA selectivity:

The quadruplex- over duplex-selectivity is evaluated through fluorescence titrations by comparison of experiments carried out with 22AG (see above) and with a short duplex-DNA: ds17, which is a 17 base-pair duplex-DNA that represents a biological sequence used in previous studies (28); the sequences of the two complementary strands are the following: [5'-CCAGTTCGTAGTAACCC-3']/[5'-GGGTTACTACGAACTGG-3']. Duplex-structure is prepared by heating the two corresponding complementary strands at 90°C for 5min in a 10mM sodium cacodylate buffer pH 7.3, 100 mM KCl followed by a slow cooling over 6hrs. Concentrations are determined by UV-Vis measurements (after thermal denaturation, 5min at 85°C) at 260nm before use.
The results are given on Fig.8.
Increasing amounts of ds17 are added onto a solution of 0.25µM of **compound 3** in cacodylate buffer (10mM sodium cacodylate + 100mM KCl) do not result in a progressive quench of the fluorescence of **compound 3** (λₑₓ = 340nm).

### c) Intra-quadruplex selectivity:

The intra-quadruplex selectivity is evaluated through fluorescence titrations by comparison of experiments carried out with 22AG (see above) and with two other quadruplex-structures: c-myc and c-kit2. The formation of these two quadruplex-DNAs is currently highly suspected in the promoter region of c-myc (see above) and c-kit (22; 29 and 30)oncogenes. The sequences are the following: c-myc: [5'-TGAGGGTGGGTAGGGTGGGTAA-3'] and c-kit2 : [(5'-CGGGCGGGCGCGAGGGAGGGG-3']; Quadruplex-structures are prepared by heating the corresponding oligonucleotide at 90°C for 5min in a 10mM sodium cacodylate buffer pH 7.2, 100 mM KCl and cooling in ice to favor the intramolecular folding by kinetic trapping. Concentrations are determined by UV-Vis measurements (after thermal denaturation, 5min at 85°C) at 260nm before use.
The results are given on Fig.9A and Fig.9B.

Increasing amounts of c-myc (left) or c-kit2 (right) are added onto a solution of 0.25µM of **compound 3** in cacodylate buffer (10mM sodium cacodylate + 100mM KCl), which result in a progressive quench of the fluorescence of **compound 3** (λₑₓ = 340nm).

### d) Summary:

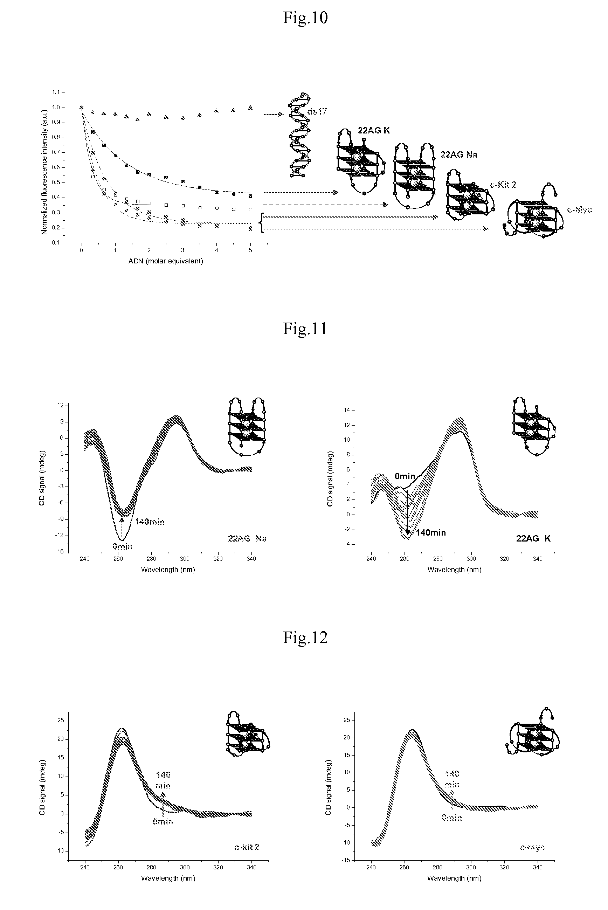

The various fluorescence titrations (λₑₓ = 340nm) are summarized in Fig.10 which gives the graphical representation:

These data indicated that **compound 3:**
- discerns very efficiently quadruplex- (22AG K, 22AG Na, c-kit 2 and c-myc) from duplex-DNA matrices (ds17), thus confirming FRET-melting results.
- interacts more efficiently and rapidly with 22AG Na, c-kit 2 and c-myc, while its interaction with 22AG K is weaker; this results are consistent with what has been observed *via* FRET-melting experiments, and represent a promising entry in the intra-quadruplex selectivity.

### Study II-4: Circular dichroism studies

Circular dichroism (CD) enables a deep study of the modification of the DNA structure upon the binding of a ligand; it thus reflects the affinity of the ligand to its target, and can also provide insight into its binding mode (Paramasivan et al, Methods, 2007, 43, 324).

### a) Quadruplex-interaction:

The quadruplex-DNA used herein is 22AG (see above), annealed both in a 10mM sodium cacodylate buffer pH 7.2, 100 mM NaCl (for 22AG Na) or KCl (for 22AG K). To a 3µM solution of 22AG in both buffers is added an excess of **compound 3** (30µM, 10 equiv.). Th results are given in Fig.11. The interaction of **compound 3** with quadruplexes is monitored as a function of time: it results in a modification of the amplitude of the CD signal of 22AG Na (left, especially at 263nm), while it reorganises completely the structure of 22AG K (right, especially at 263nm).

### b) Intra-quadruplex selectivity:

The intra-quadruplex selectivity is evaluated through CD by comparison of experiments carried out with 22AG (see above) and with two other quadruplex-structures: c-myc and c-kit2 (see above). The results are given on Fig. 11A and Fig.11B. To a 3µM solution of both c-kit2 (left) and c-myc (right) in cacodylate buffer (10mM sodium cacodylate + 100mM KCl) is added an excess of **compound 3** (30µM, 10 equiv.). The interaction of compound 3 with quadruplexes is monitored as a function of time: it results in a modification of the amplitude of the CD signal of c-kit2 (especially at 285nm) and, to a lesser extent, of c-myc (285nm).

### c) Quadruplex-structure induction:

The equilibrium between folded and unfolded forms of 22AG oligonucleotide is highly sensitive to the presence and the nature of the buffer it is used in. Corresponding results are given in Fig.12A and Fig.12B. In low cationic buffer like 10mM Tris.HCl, pH 7.2, 22AG is mostly unfolded (random coiled); it is thus possible to influence the folded <-> unfolded equilibrium by adding **compound 3.** Thus, to a 3µM solution of random coiled 22AG in Tris.HCl buffer are added increasing amounts of **compound 3** (from 0 to 7.5equiv.); it results in a deep modification of the CD signal of 22AG, with a disappearance of the random coiled-typical 257nm signal to the benefit of two novel maxima, one positive (near 290nm) and one negative (near 265nm), both being typical of the CD signature of 'anti-parallel' quadruplex-DNA structure (as 22AG Na, see above).

### d) Summary:

These data indicated that **compound 3:**
- interacts efficiently and almost spontaneously with 22AG Na, while in reacts efficiently but more slowly with 22AG K, which is consistent with fluorescence titrations and FRET-melting experiments results.
- interacts with c-myc and c-kit2, but its interaction does not modify the structure of these two quadruplexes.
- is able to shift the equilibrium between the unfolded and the folded form of 22AG toward the folded one.

### III- Biological methods and data:

### Studs III-1: Inhibition of POT 1 binding to telomeric sequence in vitro by compound 3

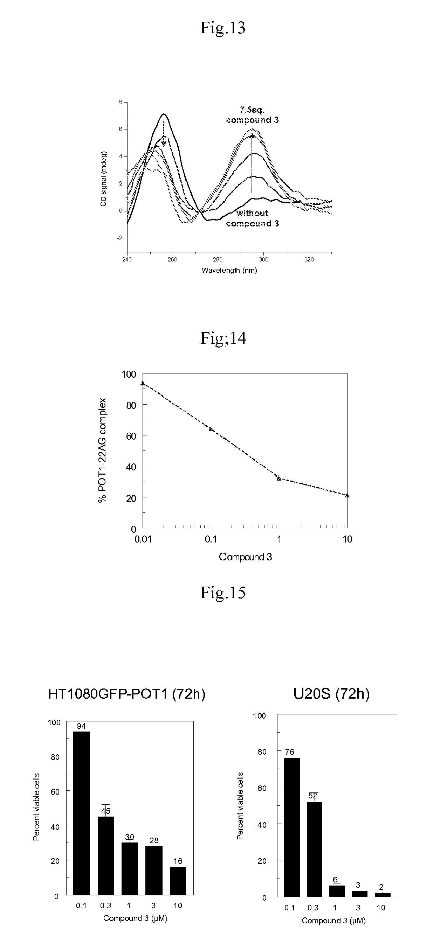

An electrophoretic mobility shift assay using hPOT1 was performed on the telomeric 22AG oligonucleotide (see above). 22AG was labeled at the 5' end with [γ-³²P]-ATP using T4 polynucletide kinase. Purified recombinant hPOT1 was produced in a baculovirus expression system. The POT1/22AG binding assay was performed in a total volume of 10µl containing 50 mM HEPES, pH 7.9, 100 mM NaCl, 0.1 mM EDTA, 4% w/v sucrose, 2% v/v glycerol, 0.1 mg/ml BSA, 0.02% w/v bromophenol blue, 30 nM hPOT1, 20 nM [α-³²P]-22AG. Different concentrations of **compound 3** (10, 1, 0.1 and 0.01 µM) were added with hPOT1 to the solution and the mixture was incubated at room temperature for 30 min. Each individual sample was separated by electroporesis on 1% agarose gel in 0.5X Tris-Borate-EDTA buffer. The gel was run at 80V for35 min, dried on whatman DE81 paper and radiaoactivity visualized by a phosphorimager (Typhoon 9210, Amersham). Analysis of the data was carried out by ImageQuant software (Amersham) and results were expressed as a percentage of the POT1-22AG complex obtained in the untreated control (defined as 100%) (see Fig. 14).

These data indicated that **compound 3** inhibits the binding of hPOT1 to telomeric 22AG sequence in a dose-dependent manner. In the experiment showed below the IC₅₀-_{POT1} for **compound 3** is equal to 300 nM.

### Studs III-2: Inhibition of cell proliferation by compound 3

The HT1080 human fibrosarcoma cell line stably transfected with pEGFP-POT1 vector (HT1080GFP-POT1) has been previously described (32) and U20S human osteosarcoma was from American Type Culture Collection (Rockville, USA). Cells were grown in Dublecco's modified Eagles's medium (Invitrogen) supplemented with 10% v/v fetal bovine serum and with 400µg/ml geneticin for HT1080GFP-POT1. Cells were plated in 6-wells culture plates on day 0 at 4.5 x10⁴ cells/well in the presence of different concentrations of **compound 3** (10, 3, 1, 0.3 and 0.1 µM), each concentration in duplicate, and cultured for further 72 hours. At day 3, cells were washed with 1X PBS and trypsinized. For each treated cell sample, the number of viable cells was determined in the presence of trypan blue. Results are given on Fig. 15A and Fig.15B. They correspond to the relative cell number in percent as compared to control untreated cells defined as 100%.

These data indicates that **compound 3** efficiently hampers the proliferation of cancer cells with an IC₅₀ comprised between 0.1 and 1µM.

### Bibliographic references

1 - Qin et al, 2008, Biochimie, 90, 1149
2- Hurley, Nature Reviews Cancer, 2002, 2, 188
3 - Neidle, et al, Nature Reviews Drug Discovery, 2002, 1, 383
4- Mergny et al, Nucleic Acids Res., 2002, 30, 839
5 - Rezler et al, Annu. Rev. Pharmacol. Toxicol., 2003, 43, 359
6 - Neidle et al, Nature Reviews Cancer, 2005, 5, 285;
7- Oganesian et al, BioEssay, 2007, 29, 155;
8 - De Cian et al, Biochimie, 2008, 90, 131; Monchaud et al, Org. Biomol. Chem., 2008, 6, 627; 9 9 - Ou et al, ChemMedChem, 2008, 3, 690)
10 - De Cian et al, Proc. Natl. Acad. Sci. U.S.A., 2007, 104, 17347
11(Tauchi et al, Oncogene, 2003, 22, 5338
12 - Tahara et al, Oncogene, 2006, 25, 1955)
13 - (Gomez et al, J. Biol. Chem., 2004, 279, 41487)
14 - (Gomez et al, J. Biol. Chem., 2006, 281, 38721)
15 - Gomez et al, Cancer Res., 2006, 66, 6908)
16 - (Doi et al, Org. Lett., 2006, 8, 4165)
17 - Tera et al, Heterocycles, 2006, 69, 505
18 - Barbieri et al, Nucleic Acids Res., 2007, 35, 3272
19 - Tera et al, Angew. Chem. Int. Ed., 2008, 47, 5557
20 - De Cian et al, Methods, 2007, 42, 183
21 - (De Cian et al, J. Am. Chem. Soc., 2007, 129, 1856)
22 - Qin et al, 2008, Biochimie, 90, 1149;
23 - Simonsson et al, Nucleic Acids Res., 1998, 26, 1167
24 - Ambrus et al, Biochemistry, 2005, 44, 2048
25 - Rangan et al, J. Biol. Chem., 2001, 276, 4640
26 - Siddiqui-Jain et al, Proc. Natl. Acad. Sci. U.S.A., 2002, 99, 11593)
27 - (26).
28 - Teulade-Fichou et at, J. Am. Chem. Soc., 2003, 125, 4732
29 - Rankin et al, J. Am. Chem. Soc., 2005, 127, 10584;
30 - Fernando et al, Biochemistry, 2006, 45, 7854
31 - Paramasivan et al, Methods, 2007, 43, 324
32 - Gomez et al , J. Biol. Chem., 2006, 281, 38721

## Claims

1. The polyheteroaryl derivatives of the invention are penta-, hexa-, hepta-, octa-, nona- and deca-heteroaryl (abbreviated hereafter as penta- to deca-heteroaryl)structurally **characterized by** a combination of heterocycle 1 (Het-1)ₐ and/or heterocycle 2 (Het-2)_{b} and/ or heterocycle 3 (Het-3)_{c} and/or heterocycle 4 (Het-4)_{d} of formulae I, II, III and IV respectively, 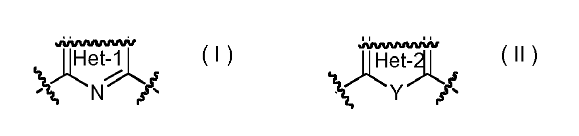 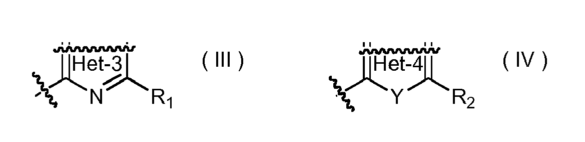 the N-oxides, the pharmaceutically acceptable addition salts, wherein
- a, b, c and d are integers from 0 to 3, the sum a + b + c + d being ≤ 10
- Y is O or S;
- Het-1 is a class of nitrogen heterocyclic-diyl ring selected in the group comprising 2,6-pyridin-diyl or 2,4-pyrimidin-diyl or 3,5-pyrazin-diyl or 2,4-(1,3,5-triazin)diyl or 3,5-(1,2,4-triazin)diyl or 2,4-oxazolin-diyl or 2,4-thiazolin-diyl , optionnally being substituted with one, two or three substituents, each independently selected from C₁₋₆alkyl ; C₁₋₄alkyloxy ; C₁₋₆alkyloxycarbonyl ; carboxy ; formyl ; halo ; cyano ; azido ; hydroxy ; nitro ; amino ; mono-or di(C₁₋₄alkyl)amino ; C₁₋₄alkylmethylamino ; morpholin-4-ylC₁₋₄alkyloxy ; piperazin-1-ylC₁₋₄alkyloxy ; 4-C₁₋₄alkylpiperazin-1-ylC₁₋₄alkyloxy ; monocyclic or bicyclic ring selected from phenyl, benzyl, naphthyl ;
- Het-2 is a class of five-membered heterocyclic-diyl ring selected in the group comprising 2,5-oxazolin-diyl or 2,5-thiazolin-diyl, or 2,5-thiophen-diyl or 2,5-furan-diyl, optionally being substituted with one or two substituents each independently selected from C₁₋₆alkyl ; C₁₋₄alkyloxy ; C₁₋₆alkyloxycarbonyl ; carboxy ; formyl ; halo ; cyano ; azido ; hydroxy ; nitro ; amino ; mono-or di(C₁₋₄alkyl)amino ; C₁₋₄alkylmethylamino ; morpholin-4-ylC₁₋₄alkyloxy ; piperazin-1-ylC₁₋₄alkyloxy ; 4-C₁₋₄alkylpiperazin-1-ylC₁₋₄alkyloxy; monocyclic or bicyclic ring selected from phenyl, benzyl, naphthyl ;
- Het-3 and/ or Het-4 constitutes the endings of the said penta- to deca-heteroaryl derivatives, wherein;
- Het-3 is a class of nitrogen heterocyclic-yl ring selected from 2-pyridyl or 2-pyrimidyl or 4-pyrimidyl or 2-pyrazyl or 2-(1,3,5)triazyl or 3-(1,2,4)triazyl or 5-(1,2,4)triazyl or 2-oxazolyl or 4-oxazolyl or 2-thiazolyl or 4-thiazolyl optionally being substituted with one, two or three substituents,each independently selected from C₁₋₆alkyl ; C₁₋₄alkyloxy ; C₁₋₆alkyloxycarbonyl ; carboxy ; formyl ; halo ; cyano ; azido; hydroxy ; nitro ; amino ; mono-or di(C₁₋₄alkyl)amino ; C₁₋₄alkylmethylamino ; morpholin-4-ylC₁₋₄alkyloxy ; piperazin-1-ylC₁₋₄alkyloxy ; 4-C₁₋₄alkylpiperazin-1-ylC₁₋₄alkyloxy ; monocyclic or bicyclic ring selected from phenyl, benzyl, naphthyl ;
- Het-4 is a class of five-membered heterocyclic-yl selected from 2-oxazolyl or 5-oxazolyl or 2-thiazolyl or 5-thiazolyl or 2-thienyl or 2-furanyl optionally being substituted with one or two substituents each independently selected from
- C₁₋₆alkyl ; C₁₋₄alkyloxy ; C₁₋₆alkyloxycarbonyl ; carboxy ; formyl ; halo ; cyano ; azido ; hydroxy ; nitro ; amino ; mono-or di(C₁₋₄alkyl)amino ;
- C₁₋₄alkylmethylamino ; morpholin-4-ylC₁₋₄alkyloxy; piperazin-l-ylC₁₋₄alkyloxy; 4-C₁₋₄alkylpiperazin-l-ylC₁₋₄alkyloxy ; monocyclic or bicyclic ring selected from phenyl, benzyl, naphthyl ;
- R₁ and R₂, are each independently selected from hydrogen; C₁₋₆alkyl ; C₁₋₄alkyloxy ; C₁₋₆alkyloxycarbonyl ; carboxy ; formyl ; halo ; cyano ; azido ; hydroxy ; nitro ; amino ; mono-or di(C₁₋₄alkyl)amino ; C₁₋₄alkylmethylamino ; monocyclic or bicyclic ring selected from phenyl, benzyl, naphthyl optionally being substituted with one, two or three substituents each independently selected from halo, hydroxy, C₁₋₄alkyl, C₁₋₄alkyloxy, morpholin-4-yl C₁₋₄alkyloxy, piperazin-1-ylC₁₋₄alkyloxy, 4-C₁₋₄alkylpiperazin-1-yl C₁₋₄alkyloxy or phenyl.

2. The derivatives of claim 1, wherein Het-1 is linked to two Het-2.

3. The derivatives of claim 2, wherein Het-1 is a pyridyl and Het-4 is an oxazolyl, substituted by a pyridyl or pyridyl-oxazolyl group.

4. The derivatives of claim 1, wherein Het-2 is linked to two Het-1.

5. The derivatives of claim 4, wherein Het-2 is an oxazolyl and Het-3 is a pyridyl-oxazolyl group.

6. The use of the derivatives according to anyone of claims 1 to 5 as drugs.

7. Pharmaceutical compositions comprising an effective amount of at least one derivative according to anyone of claims 1-5 in combination with a pharmaceutically acceptable carrier.

8. The pharmaceutical compositions of claim 7, under forms suitable for an administration by the oral or parenteral route.

9. The pharmaceutical compositions of claim 7 or 8, for treating cancer and infectious diseases, such as malaria.

10. The use of the derivatives according to anyone of claims 1-5, for making a drug for treating cancer or infectious diseases.

11. A method for preparing polyheteroaryl derivatives of anyone of claims 1 to 5, comprising
• reacting X1-(di- or tri- or tetra- heteroaryl-1,4)-X2
• with a (mono- or di- or tri- or tetra- heteroaryl-1,4)-X3
wherein
- Heteroaryl-1,4 is a combination of Het-1 and/or Het-2 and/or Het-3 and/or Het-4 which are as defined above,
- X1 and X2, identical or different, represent an hydrogen or halogen or triflate group,
- X3 represents an halogen or zinc halogenide or trialkyltin or boronate group,
under conditions giving the desired polyheteroaryl derivatives.
